(19) Europäisches Patentamt
European Patent Office
Office européen des brevets

(11) Veröffentlichungsnummer: **0 308 746 A2**

(12) # EUROPÄISCHE PATENTANMELDUNG

(21) Anmeldenummer: **88114761.5**

(51) Int. Cl.4: **C07C 127/22 , A01N 47/34**

(22) Anmeldetag: **09.09.88**

(30) Priorität: **19.09.87 DE 3731561**

(43) Veröffentlichungstag der Anmeldung: **29.03.89 Patentblatt 89/13**

(84) Benannte Vertragsstaaten: **AT BE CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Seppelt, Wolfgang, Dr.**
**Stettiner Strasse 1a**
**D-6712 Bobenheim-Roxheim(DE)**
Erfinder: **Hofmeister, Peter, Dr.**
**Bernard-Humblot-Strasse 12**
**D-6730 Neustadt(DE)**
Erfinder: **Kuenast, Christoph, Dr.**
**Salierstrasse 2**
**D-6701 Otterstadt(DE)**

(54) **N-Benzoyl-N'-(2,3-dichlor-4-phenoxy)-phenyl-harnstoffe.**

(57) N-Benzoyl-N'-(2,3-dichlor-4-phenoxy)-phenyl-harnstoffe der allgemeinen Formel I

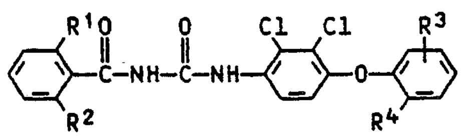

I

in der die Substituenten
$R^1$ Halogen,
$R^2$ Wasserstoff oder Halogen,
$R^3$ Halogen oder $C_1$-$C_4$-Halogenalkoxy,
$R^4$ Wasserstoff oder Halogen bedeuten,
deren Herstellung und Verwendung zur Bekämpfung von Schädlingen.

EP 0 308 746 A2

## N-Benzoyl-N′-(2,3-dichlor-4-phenoxy)-phenyl-harnstoffe

Die vorliegende Erfindung betrifft neue N-Benzoyl-N′-(2,3-dichlor-4-phenoxy)-phenyl-harnstoffe der allgemeinen Formel I

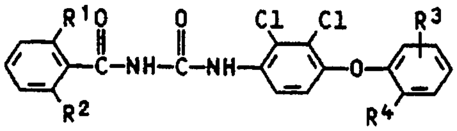

(I)

in der die Substituenten folgende Bedeutung haben:

$R^1$ Halogen,
$R^2$ Wasserstoff oder Halogen,
$R^3$ Halogen oder $C_1$-$C_4$-Halogenalkoxy,
$R^4$ Wasserstoff oder Halogen.

Außerdem betrifft die Erfindung die Herstellung der Verbindungen I, Schädlingsbekämpfungsmittel, die die Verbindungen I als Wirkstoffe enthalten, sowie ein Verfahren zur Bekämpfung von Schädlingen.

Aus der DE-A-25 31 202, der DE-A-32 32 265 und der DE-A-33 09 987 sind N-Benzoyl-N′-phenoxyphenylharnstoffe zur Bekämpfung von Schädlingen bekannt, die jedoch nicht im N-Phenylteil in 2,3-Stellung chloriert sind. Die Verbindungen des Standes der Technik lassen in ihrer Wirkung zu wünschen übrig.

Der Erfindung lag daher die Aufgabe zugrunde, neue Harnstoffderivate mit verbesserter Wirkung bereitzustellen.

Demgemäß wurden die eingangs definierten neuen N-Benzoyl-N′-(2,3-dichlor-4-phenoxy)-phenyl-harnstoffe I, Verfahren zu deren Herstellung, Schädlingsbekämpfungsmittel, die die Verbindungen I als Wirkstoffe enthalten, sowie ein Verfahren zur Bekämpfung von Schädlingen mit N-Benzoyl-N′-(2,3-dichlor-4-phenoxy)-phenyl-harnstoffen I gefunden.

Die Verbindungen I sind nach folgenden Methoden erhältlich:

a) durch Umsetzung eines Benzoylisocyanates der allgemeinen Formel II

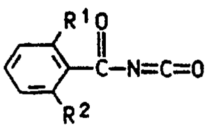

(II)

mit einem (2,3-Dichlor-4-phenoxy)-anilin der allgemeinen Formel III

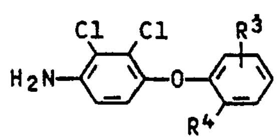

(III)

oder

b) durch Umsetzung eines Benzamides der allgemeinen Formel IV

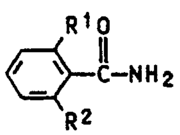

(IV)

mit einem (2,3-Dichlor-4-phenoxy)-phenylisocyanat der allgemeinen Formel V

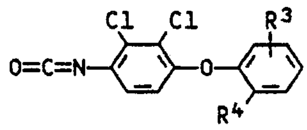 (V).

Die Umsetzung a) verläuft bei Temperaturen von 0 bis 120 °C, vorzugsweise 20 bis 80 °C und besonders bevorzugt bei 20 bis 60 °C, sowie bei Drücken zwischen 1 und 10 bar, vorzugsweise bei Normaldruck. Da die Reaktion unter Wärmeentwicklung (exotherm) verläuft, kann es von Vorteil sein, eine Kühlmöglichkeit vorzusehen.

Zweckmäßigerweise wird das (2,3-Dichlor-4-phenoxy)-anilin der Formel III in einem Lösungs- oder Verdünnungsmittel vorgelegt unter anschließender Zugabe des Benzoylisocyanats der Formel II. Die Reaktion ist im allgemeinen nach 2 Stunden beendet.

Die Benzoylisocyanate II sind bekannt oder nach bekannten Methoden herstellbar (J. Org. Chem. 28, 1805-1811 (1963) oder J. Agr. Chem. 21, 3488f (1973). Die (2,3-Dichlor-4-phenoxy)-aniline III sind gleichfalls bekannt oder nach bekannten Methoden herstellbar (Houben/Weyl, Methoden der Organischen Chemie, Bd. XI/1, 360 ff. (1975). Die zugrunde liegenden Nitrobenzole und deren Herstellung sind aus Barry et al., Pr. Irish Acad. 53B, 6166-82 (1950) bekannt.

Die Umsetzung b) wird gegebenenfalls in Gegenwart eines Katalysators, bei Temperaturen von 0 bis 140 °C, vorzugsweise zwischen 60 und 100 °C, sowie bei Drücken zwischen 1 und 10 bar, vorzugsweise bei Normaldruck, durchgeführt. Die Reaktionsdauer liegt im allgemeinen zwischen 2 und 6 Stunden.

Als Katalysatoren eignen sich beispielsweise organische Basen, wie Triethylamin, Pyridin, 4-N,N-Dimethylaminopyridin oder Alkylmetallverbindungen, wie Dibutylzinndiacetat,

Die Benzamide IV sind bekannt oder nach bekannten Methoden (z.B. Houben-Weyl, Methoden der organischen Chemie, Bd. E 5.2, S. 934 ff, Georg-Thieme Verlag, 1985) erhältlich. Die (2,3-Dichlor-4-phenoxy)-phenylisocyanate V sind z.T. bekannt oder sie können nach bekannten Methoden (z.B. Houben-Weyl, Methoden der organischen Chemie, Bd. E 4, S. 738 ff, Georg Thieme Verlag, 1983) erhalten werden.

Üblicherweise setzt man die Ausgangsstoffe II und III bzw. IV und V in stöchiometrischem Verhältnis ein. Ein Überschuß der einen oder anderen Komponente kann in Einzelfällen aber durchaus vorteilhaft sein.

Die Umsetzungen a) und b) (vgl. auch DE-A-21 23 236) werden zweckmäßigerweise in einem Lösungs- oder Verdünnungsmittel ausgeführt. Hierzu sind beispielsweise geeignet: aliphatische und aromatische, gegebenenfalls chlorierte Kohlenwasserstoffe, wie Petrolether, Benzol, Toluol, Xylol, Benzin, Dichlormethan, Chloroform, Tetrachlormethan, 1,2-Dichlormethan und Chlorbenzol, Ether oder Ester wie Diethyl- und Di-n-Butylether, Methyl-tert.-butylether, Tetrahydrofuran, Dioxan und Essigsäureethylester; Ketone, beispielsweise Aceton, Methylethylketon und Methylisopropylketon; ferner Nitrile, wie Acetonitril und Propionitril; aprotische dipolare Lösungsmittel, wie Dimethylformamid, Dimethylsulfoxid oder Pyridin. Auch Gemische dieser Stoffe können als Lösungs- oder Verdünnungsmittel verwendet werden.

Die neuen Verbindungen der Formel I fallen teilweise in Form von Ölen an, die sich durch längeres Erwärmen unter vermindertem Druck auf mäßig erhöhte Temperatur ("Andestillieren") von den letzten flüchtigen Anteilen befreien und auf diese Weise reinigen lassen. Erhält man die Verbindungen der Formel I in kristalliner Form, so kann ihre Reinigung durch Umkristallisation erfolgen.

Im einzelnen haben die Substituenten in Formel I folgende Bedeutungen:

$R^1$ - Halogen, wie Fluor, Chlor, Brom und Jod, bevorzugt Fluor und Chlor,

$R^2$ - Wasserstoff,

- Halogen, wie Fluor, Chlor, Brom und Jod, bevorzugt Fluor und Chlor,

$R^3$ - Halogen, bevorzugt Fluor, Chlor und Brom $C_1$-$C_4$-Halogenalkoxy, bevorzugt $C_1$-$C_2$-Fluor- und Chloralkoxy, besonders bevorzugt Fluor und Chlor substituiertes Methoxy sowie Fluormethoxy, Difluormethoxy, Trifluormethoxy, Chlormethoxy, Dichlormethoxy, Trichlormethoxy, Chlor-fluormethoxy, Dichlorfluormethoxy und Chlordifluormethoxy,

$R^4$ - Wasserstoff,

- Halogen, bevorzugt Fluor, Chlor und Brom

Die N-Benzoyl-N′-(2,3-dichlor-4-phenoxy)-phenyl-harnstoffe der allgemeinen Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Im Gegensatz zu den meisten bisher bekannten Wirkstoffen, die als Kontakt-oder Fraßgifte die Tiere töten, lähmen oder vertreiben, wirken die Verbindungen der Formel I entwicklungsstörend auf den

tierischen Organismus. Bei Insekten wird beispielsweise die Umwandlung zur Imago, die Ablage von entwicklungsfähigen Eiern und die Entwicklung von abgelegten normalen Eiern gestört und dadurch die Generationsfolge unterbrochen. Für Wirbeltiere sind die erfindungsgemäßen Wirkstoffe praktisch ungiftig. Die Verbindungen der Formel I werden überdies leicht zu Stoffen abgebaut, die in der natürlichen Umgebung vorkommen und von Mikroorganismen weiter zerlegt werden. Die Gefahr einer Kumulation ist deshalb ausgeschlossen. Sie können demgemäß unbedenklich zur Bekämpfung von Schädlingen bei Tieren, Pflanzen und Vorräten eingesetzt werden.

Zu den schädlichen Insekten gehören aus der Ordnung der Schmetterlinge Lepidoptera) beispielsweise Plutella maculipennis (Kohlschabe), Leucoptera coffeella (Kaffeemotte), Hyponomeuta malinellus (Apfelbaum gespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria mellonella (Wachsmotte), Malacosoma neustria (Ringelspinner), Dendrolimus pini (Kiefernspinner), Thaumatopoea ptiyocampa (Pinienprozessionsspinner), Phalera bucephala (Mondfleck), Cheimatobia brumata (Kleiner Frostspanner), Hibernia defoliaria (Großer Frostspanner), Bupalus piniarius (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweißling);
aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agriotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varivestis (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagai (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-puncta (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Linierter Blattrandkäfer), Ortiorrhynchus sulcatus (Gefurchter Lappenrüßler), Otiorrhynchus ovatus (Erdbeerwurzelrüßler), Hylobius abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrhynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefurchter Waldgärtner);
aus der Ordnung der Zweiflügler (Diptera) beispielsweise Lycoria pectoralis, Mayetiola destructor (Hessenfliege), Basineura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus oleae (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hysocyami (Rübenfliege), Anopheles maculipennis, Culex pipiens, Aedes aegypti (Gelbfiebermücke), Aedes vexans, Tabanus bovinus (Rinderbremse), Tipula paludosa (Wiesenschnake), Musca domestica (Stubenfliege), Fannia canicularis (Kleine Stubenfliege), Muscina stabulans, Glossina morsitans (Tsetse-Fliege), Oestrus ovis, Chrysomya macellaria, Chrysomya hominivorax, Lucilia cuprina, Lucilia sericata, Hypoderma lineata;
aus der Ordnung der Hautflügler (Hymenoptera) beispielsweise Athalia rosae (Rübenblattwespe), Hoplocampa minuta (Pflaumensägewespe), Monomorium pharaonis (Pharaoameise), Solenopsis geminata (Feuerameise), Atta sexdens (Blattschneiderameise);
aus der Ordnung der Wanzen (Heteroptera) beispielsweise Nezara viridula (Grüne Reiswanze), Eurygaster integriceps (Asiatische Getreidewande), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze);

aus der Ordnung der Pflanzensauger (Homoptera) beispielsweise Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlaus), Aphidula nasturtii (Kreuzdornblattlaus), Cerosipha gossypii (Gurkenblattlaus), Sappaphis mali (Rosige Apfellaus), Sappaphis mala (Mehlige Birnblattlaus), Dysaphis radicola (Mehlige Apfelfaltenlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Dysaulacorthum pseudosolani (Gefleckte Kartoffellaus), Acyrthosiphon onobrychis (Grüne Erbsenalaus), Macrosiphon rosae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus);
aus der Ordnung der Termiten (Isoptera) beispielsweise Reticulitermes lucifugus, Calotermes flavicollis, Leucotermes flavipes, Termes natalensis;
aus der Ordnung der Geradflügler (Orthoptera) beispielsweise Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrecke), Melanoplus spretus (Felsengebirgsheuschrecke), Melanoplus femur-rubrum (Rotbeinige Heuschrecke), Blatta orientalis (Küchenschabe), Blattella germanica (Deutsche Schabe), Periplaneta americana (Amerikanische Schabe), Blabera gigantes (Riesenschabe).

Zur Klasse der Arachnoidea gehören Spinntentiere (Acarina) beispielsweise Ixodes ricinus (Holzbock), Ornithodorus moubata, Amblyomma americanum, Dermacentor silvarum, Boophilus microplus, Tetranychus telarius, Tetranychus pacificus, Paratetranychus pilosus, Bryobia praetiosa.

Zur Klasse der Nematoden zählen beispielsweise Wurzelgallennematoden, z.B. Meloidogyne incognita, Meloidogyne hapla, Meloidogyne javanica, Zysten bildende Nematoden, z.B. Globodera rostochiensis, Heterodera schachtii, Heterodera avenae, Heterodera glycinae, Heterodera trifolii, Stock- und Blattälchen, z.B. Ditylenchus dipsaci, Ditylenchus destructor, Pratylenchus neglectus, Pratylenchus penetrans, Pratylenchus goodeyi, Paratylenchus curvitatus sowie Tylenchorhynchus dubius, Tylenchorhynchus claytoni, Rotylenchus robustus, Heliocotylenchus multicinctus, Radopholus similis, Belonolaimus longicaudatus, Longidorus elongatus, Trichodorus primitivus.

Die Wirkstoffe können als solche, in Form ihrer Formulierungen oder den daraus bereiteten Anwendungsmöglichkeiten, z.B. in Form direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbare Pulver (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkali, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Aklylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolylglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 5 Gew.-Teile der Verbindung Nr.1 werden mit 95 Gew.-Teilen feinteiligem Kaolin innig vermischt. Man erhält auf diese Weise ein Stäubemittel, das 3 Gew.-% des Wirkstoffs enthält.

II. 30 Gew.-Teile der Verbindung Nr.7 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gew.-Teile der Verbindung Nr.11 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.

IV. 20 Gew.-Teile der Verbindung Nr.17 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.

V. 80 Gew.-Teile der Verbindung Nr.18 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%.

Die Wirkstoffkonzentrationen in den anwendungsfertigen Zubereitungen können in größeren Bereichen variiert werden.

Im allgemeinen liegen sie zwischen 0,0001 und 10 Gew.%, vorzugsweise zwischen 0,01 und 1 Gew.%. Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff beträgt unter Freilandbedingungen 0,001 bis 10, vorzugsweise 0,02 bis 2 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Herstellungsbeispiel

N-2,6-Difluorbenzoyl-N′-2,3-dichlor-4-(4-brom-2-fluorphenoxy)-phenyl-harnstoff (Verbindung Nr. 17)

21 g (0,11 Mol) 2,3,4-Trichlornitrobenzol, 21,5 g (0,11 Mol) 4-Brom-2-fluor-phenol und 31 g $K_2CO_3$ werden in 150 ml Dimethylformamid 24 Stunden bei 120 °C gerührt. Nach dem Abkühlen wird filtriert und das Lösungsmittel i. Vak. abdestilliert. Man erhält 41 g (97 %) öliges Rohprodukt, das direkt weiterverarbeitet wird.

38 g (0,1 Mol) des zuvor erhaltenen 2,3-Dichlor-4-(4-brom-2-fluor-phenoxy)-anilins werden in 300 ml Tetrahydrofuran in Gegenwart von 4 g Raney-Nickel bei Raumtemperatur unter einem Druck von 10 bar 5 Stunden hydriert. Nach dem Abfiltrieren des Katalysators und Abdestillieren des Lösungsmittels bleibt 35 g

(99 %) des erstarrten Produktes zurück.

Zu 7 g (0,02 Mol) des zuvor erhaltenen 2,3-Dichlor-4-(-4-brom-2-fluro-phenoxy)-anilins in 40 ml Tetrahydrofuran tropft man bei Raumtemperatur 3,7 g (0,02 Mol) 2,6-Difluorbenzoylisocyanat in 80 ml Toluol. Anschließend wird 3 Stunden bei 45° C gerührt. Nach Entfernung des Lösungsmittels i.Vak. wird der Rückstand in Petrolether aufgenommen und abgesaugt. Man erhält 7,9 g (74 %) N-2,6-Difluorbenzoyl-N′-2,3-dichlor-4(4-brom-2-fluor-phenoxy)-phenyl-harnstoff (Verbindung Nr. 17);
Smp.: 167° C

Die in der nachstehenden Tabelle aufgeführten Verbindungen I können nach dem erfindungsgemäßen Verfahren aus entsprechenden Vorprodukten ohne weiteres erhalten werden und lassen eine gleichartige Wirkung erwarten.

Tabelle

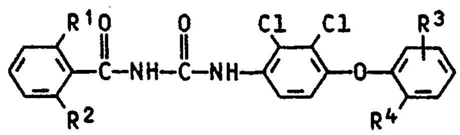

(I)

| Verbindung Nr. | $R^1$ | $R^2$ | $R^3$ | $R^4$ | Fp. [°C] |
|---|---|---|---|---|---|
| 1 | F | F | 4-F | H | 175 |
| 2 | Cl | H | 4-F | H | 146 |
| 3 | Cl | Cl | 4-F | H | |
| 4 | Cl | H | 4-Cl | H | |
| 5 | F | F | 4-Cl | Cl | |
| 6 | F | H | 4-F | H | 142 |
| 7 | F | F | 4-F | Cl | 151 |
| 8 | F | F | 4-Cl | H | |
| 9 | F | F | 4-Br | H | |
| 10 | F | F | 4-F | Br | 162 |
| 11 | F | F | 3-F | H | 182 |
| 12 | Cl | H | 3-F | H | 122 |
| 13 | Cl | H | 5-F | Cl | |
| 14 | Cl | H | 5-Cl | Cl | |
| 15 | F | F | 5-Cl | Cl | |
| 16 | Cl | H | 4-Br | F | 158 |
| 17 | F | F | 4-Br | F | 167 |
| 18 | F | F | 4-$OCF_3$ | H | 155 |
| 19 | Cl | H | 4-$OCF_3$ | H | 161 |
| 20 | Cl | H | 4-$OCF_3$ | Cl | |

## Anwendungsbeispiele

In den folgenden Beispielen wurden die erfindungsgemäßen Verbindungen bzw. sie enthaltende Mittel hinsichtlich ihrer Wirkung auf Schädlinge mit den nachstehenden Verbindungen des Standes der Technik

verglichen.

A: 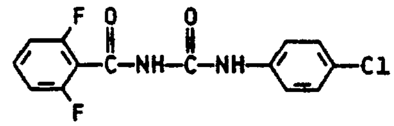

bekannt aus DE-A-21 23 236 als Verbindung des Anspruchs Nr. 69

B: 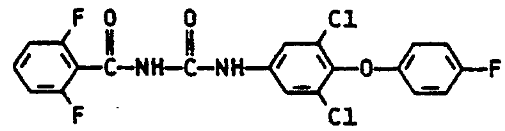

Verbindung Nr. 8 aus DE-A 32 32 265

Die Konzentrationen, bei denen die untersuchten Verbindungen eine 100 %ige Mortalität bzw. Hemmung bewirken, sind die jeweiligen Minimalkonzentrationen. Die Reinheit der Vergleichsverbindungen A und B unterschied sich nicht wesentlich von der der erfindungsgemäßen Verbindungen und betrug jeweils größer 95 %.

Beispiel A

Zuchtversuch mit Aedes aegypti (Gelbfiebermücken)

Bei 25°C werden in einem 250 ml Plastikbecher 200 ml Leitungswasser mit der Wirkstoffaufbereitung versetzt und anschließend mit 20 bis 30 Moskitolarven im dritten bis vierten Larvenstadium besetzt. Während der Versuchsdauer wird einmal mit einem gepulverten, käuflichen Zierfischfutter (Tetramin®) gefüttert. Beobachtet werden Verpuppung und Schlupf der Imagines. Dies erfolgt nach ca. 10 bis 12 Tagen.

In diesem Versuch lag die mortale Dosis der Verbindung Nr. 11, 17 und 18 bei 0,002 ppm; bei der Vergleichsverbindung A bei 0,004 ppm; bei der Vergleichsverbindung B wurden 100 % Mortalität bei 0,04 ppm beobachtet, während die strukturnächste Verbindung 1 bereits bei 0,004 ppm eine 100 %ige Mortalitätsrate bewirkte.

Beispiel B

Musca domestica (Stubenfliege), Zuchtversuch

Als Versuchsgefäße dienen 50 ml Penicillingläser. Diese werden mit je 4,75 ml Nährbrei beschickt und mit 0,25 ml wäßriger Wirkstoffaufbereitung versetzt.

In einem Becherglas schwemmt man ca. 10.000 einen Tag alte Fliegeneier in ca. 100 ml Leitungswasser auf. Aus dieser Suspension überträgt man mittels einer Pipette je einen Tropfen in ein Glas.

Der fertige Versuchsansatz wird mit einem Wattepfropfen verschlossen und bei 23 bis 24°C gelagert. Am 5. Tag erfolgt die Auswertung.

In diesem Versuch zeigte Verbindung 1 bei einer Konzentration von 1 ppm eine Mortalitätsrate von 80 % während das Vergleichsmittel B bei dieser Konzentration keine Wirkung aufwies und erst bei der zweifachen Konzentration 100 % Mortalität bewirkte.

Beispiel C

Entwicklungshemmung von Heliothis virescens auf behandeltem Nährboden

100 g Standard-Nährbodens für Heliothis werden in 250 ml-Becher gefüllt und warm mit der wäßrigen

Wirkstoffaufbereitung sorgfältig gemischt. Nach Erkalten belegt man jedes Gefäß mit 10 Raupen im dritten Larvenstadium (1,5 bis 1,8 cm) und lagert sie bei 23° C. Beurteilt wird Verpuppung und Schlupf der Falter.

Bei diesem Versuch wurde mit 0,1 ppm der Verbindung Nr. 11 eine Mortalitätsrate von 80 %, mit 0,1 ppm der Verbindung 17 eine Mortalitätsrate von 90 % und mit 4 ppm der Vergleichsverbindung A eine Mortalitätsrate von 90 % erzielt.

Beispiel D

Entwicklungshemmung von Agrotis ypsilon (Edraupe) auf behandeltem Nährboden

100 g Standard-Nährbodens für Agrotis werden in 250 ml-Becher gefüllt und warm mit der wäßrigen Wirkstoffaufbereitung sorgfältig gemischt. Nach Erkalten belegt man jedes Gefäß mit Raupen im dritten Larvenstadium (1,5 bis 1,8 cm) und lagert sie bei 23° C. Beurteilt wird Verpuppung und Schlupf der Falter. Pro Konzentration werden 2 Becher mit zusammen 20 Raupen angesetzt.

Bei diesem Versuch lag die mortale Dosis der Verbindung Nr. 7, 11, 17 und 18 bei 0,1 ppm. Mit 1 ppm der Verbindung Nr. 19 oder 4 ppm der Verbindung Nr. 1 erzielte man eine Mortalitätsrate von 90 %. Die Vergleichsverbindung A zeigte bei 10 ppm eine Mortalitätsrate von 80 %.

Beispiel E

Zuchtversuch mit Dysdercus intermedius (Baumwollwanze)

Petrischalen von 10 cm Durchmesser werden mit 1 ml der acetonischen Wirkstofflösung ausgekleidet. Nach Verdunsten des Lösungsmittels besetzt man die Schalen mit je 20 Larven des vorletzten Stadiums.

Nach 24 Stunden überführt man die überlebenden Tiere in 1 l-Weckgläser, die 200 g sterilen Quarzsand (Korngröße 0 bis 3 mm) enthalten. Dieser Sand wurde vor Versuchsbeginn mit 25 ml der wäßrigen Wirkstoffaufbereitung angegossen.

Als Futter bietet man gequollene Baumwollsamen, die wöchentlich gewechselt werden. Ebenfalls wöchentlich feuchtet man den Sand mit reinem Wasser an.

Die Versuchstemperatur beträgt 25 bis 27° C. Die Beobachtungszeit erstreckt sich bis zum Schlüpfen der Eier in den Kontrollen. Beurteilt wird die Mortalitätsrate.

In diesem Versuch zeigten Verbindungen 1 und Vergleichsverbindung B jeweils bei 4 ppm 100 % Mortalität.

Beispiel F

Zuchtversuch mit Ostrinia nubilalis (Maiszünsler)

Die Zucht erfolgt auf einem Nährboden folgender Zusammensetzung:

515 g Maismehl
130 g Weizenkeime
137 g Bierhefe
18 g Ascorbinsäure
10 g Cellulosepulver
5 g Nipagin
20 g Wessons Salz
20 ml Vitaminlösung
80 g Agar
3100 ml Wasser

Je 50 ml füllt man in 100 ml Plastikbecher und mischt die wäßrige Wirkstoffaufbereitung sorgfältig unter.

Nach Erkalten belegt man jedes Gefäß mit 4 Raupen (L 3). Pro Konzentration werden 5 Gefäße

angesetzt.

Die Beobachtung erstreckt sich bis zum Schlüpfen der Falter.

In diesem Versuch bewirkte Verbindung 1 eine Mortalitätsrate von 100 % bei einer Wirkstoffkonzentration von 20 ppm, während die Vergleichsverbindung B bei dieser Konzentration keine Aktivität zeigte (0 % Mortalität).

**Ansprüche**

1. N-Benzoyl-N′-(2,3-dichlor-4-phenoxy)-phenyl-harnstoffe der allgemeinen Formel I

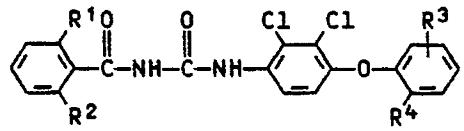

(I)

in der die Substituenten folgende Bedeutung haben:

$R^1$ Halogen,

$R^2$ Wasserstoff oder Halogen,

$R^3$ Halogen oder $C_1$-$C_4$-Halogenalkoxy,

$R^4$ Wasserstoff oder Halogen.

2. N-Benzoyl-N′-(2,3-dichlor-4-phenoxy)-phenyl-harnstoffe der Formel I nach Anspruch 1, in der die Substituenten folgende Bedeutung haben:

$R^1$ Fluor oder Chlor,

$R^2$ Wasserstoff, Fluor oder Chlor,

$R^3$ Fluor, Chlor, Brom oder Trifluormethoxy,

$R^4$ Wasserstoff, Fluor, Chlor oder Brom.

3. Verfahren zur Herstellung von N-Benzoyl-N′-(2,3-dichlor-4-phenoxy)-phenyl-harnstoffen der allgemeinen Formel I gemäß Anspruch 1, dadurch gekennzeichnet, daß man

a) ein Benzoylisocyanat der allgemeinen Formel II

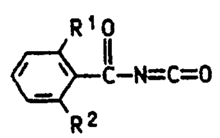

(II)

mit einem (2,3-Dichlor-4-phenoxy)-anilin der allgemeinen Formel III

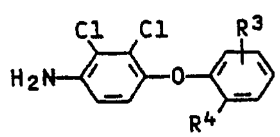

(III)

oder

b) ein Benzamid der allgemeinen Formel IV

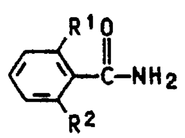

(IV)

mit einem (2,3-Dichlor-4-phenoxy)-phenylisocyanat der allgemeinen Formel V

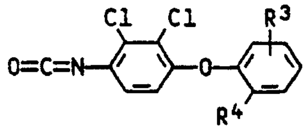

(V)

in an sich bekannter Weise umsetzt.

4. Schädlingsbekämpfungsmittel, enthaltend einen N-Benzoyl-N′-(2,3-dichlor-4-phenoxy)-phenyl-harnstoff der Formel I gemäß Anspruch 1 neben üblichen Trägerstoffen.

5. Schädlingsbekämpfungsmittel gemäß Anspruch 4, dadurch gekennzeichnet, daß es 0,1 bis 95 Gew.% eines N-Benzoyl-N′-(2,3-dichlor-4-phenoxy)-phenyl-harnstoffes I enthält.

6. Verfahren zur Bekämpfung von Schädlingen, dadurch gekennzeichnet, daß man die Schädlinge und/oder die von Schädlingen freizuhaltenden Flächen und/oder Räume mit einer für Schädlinge wirksamen Menge eines N-Benzoyl-N′-(2,3-dichlor-4-phenoxy)-phenyl-harnstoffes der Formel I gemäß Anspruch 1 behandelt.